Europäisches Patentamt

European Patent Office

Office européen des brevets

(19)

(11) Veröffentlichungsnummer: **0 266 303 B1**

## (12) EUROPÄISCHE PATENTSCHRIFT

(45) Veröffentlichungstag der Patentschrift: **29.07.92**

(51) Int. Cl.⁵: **A61K 31/565**, A61K 31/575

(21) Anmeldenummer: **87730107.7**

(22) Anmeldetag: **15.09.87**

(54) **Antigestagene zur Hemmung der uterinen Prostaglandinsynthese.**

(30) Priorität: **26.09.86 DE 3633244**

(43) Veröffentlichungstag der Anmeldung:
**04.05.88 Patentblatt 88/18**

(45) Bekanntmachung des Hinweises auf die
Patenterteilung:
**29.07.92 Patentblatt 92/31**

(84) Benannte Vertragsstaaten:
**AT BE CH DE ES FR GB GR IT LI LU NL SE**

(56) Entgegenhaltungen:

**Comparative pharmacological studies on
new progesterone-antagonists etc., Elger et
al., Nichidoku-Iho, vol. 33, no. 4, 1990**

**V.R. PICKLES et al.: "Prostaglandins in endometrium and menstrual fluid from normal
and dysmenorrhoeic subjects"**

**BR. J. OBSTET. GYNECOL., Band 83, Nr. 3,
Mai 1976, Seiten 337-341 E.A.WILLMAN et al.:
"Studies in the involvement of prostaglandins in uterine symptomatology and pathology"**

(73) Patentinhaber: **SCHERING AKTIENGESELL-
SCHAFT Berlin und Bergkamen
Müllerstrasse 170/178 Postfach 65 03 11
W-1000 Berlin 65(DE)**

(72) Erfinder: **Elger, Walter, Dr.
Schorlemer Allee 12 B
W-1000 Berlin 33(DE)**
Erfinder: **Fähnrich, Marianne, Dipl.-Biologin
Letteallee 48
W-1000 Berlin 51(DE)**
Erfinder: **Beier, Sybille, Dr. Dipl.-Biologin
Uhlandstrasse 121
W-1000 Berlin 31(DE)**
Erfinder: **Chwalisz, Krzysztof, Dr.
Luzerner Strasse 1
W-1000 Berlin 45(DE)**

J. ENDROCR., Band 77, 1978, Seiten 361-371, GB J.B. MAATHUIS et al.: "Concentrations of prostaglandins F2 alpha and E2 in the endometrium throughout the human menstrual cycle, after the administration of clomiphene or an oestrogenprogestogen pill and in early pregnancy"

AM. J. OBSTET. GYNAECOL., Band 157 (4Pt2), Oktober 1987, Seiten 1065-1074 W-EIGER et al.: "Endometrial and myometrial effects of progesterone antagonists in pregnant guinea pigs"

ACTA ENDROCRINOL (Suppl.), Band 114, Juni 1987, Seite 126 M. FÄNRICH: "The influence of progesterone antagonists on PG-liberation in different uterine compartments"

ACTA ENDOCRINOL (Suppl.), Band 114, Juni 1987, Seiten 7, 8 J. NEULEN et al.: "Influence of the antigestagens ZK 98.299 and ZK 98.734 on the production of prostaglandin F2alpha (PG F2alpha) and prostaglandin E2(PG E2) in human endometrium in vitro"

J. OBSTET GYNAECOL. BR. COMMONW. Band 72, 1965, Seiten 185-192

## Beschreibung

Die Erfindung betrifft die Verwendung von Antigestagenen für die Herstellung von Arzneimitteln zur Hemmung der uterinen Prostaglandinsynthese, insbesondere zur Therapie und Prophylaxe dysmenorrhoeischer Beschwerden.

Wirksame Antigestagene sind zum Beispiel aus der europäischen Patentschrift 0057115 (Roussel-Uclaf) bekannt.

Besonders eingehend untersucht wurde das als RU 486 bezeichnete 11$\beta$-[4-(N,N-Dimethylamino)-phenyl]-17$\beta$-hydroxy-17$\alpha$-propinyl-4,9(10)-estradien-3-on. Angaben uber den Wirkungsmechanismus von RU 486 finden sich in "Human Reproduction", Vol. 1 (1986), 107-110. Danach bewirkt das Antigestagen eine Blockierung der Progesteronaktivität und einen Anstieg der Prostaglandinbildung, wobei das Prostaglandin die Uteruskontraktilität stimuliert. Mit RU 486 wurden bereits klinische Untersuchungen zur Beendigung einer Schwangerschaft durchgeführt.

Prostaglandinen kommt eine Schlüsselrolle für die normale und pathologische Menstruation zu: Sie werden in Beziehung gesehen zu Veränderungen der Blutversorgung der Uterusschleimhaut und zu den wehenähnlichen Kontraktionen des Uterus während der Menstruation. Eine gesteigerte PG-Freisetzung und gesteigerte Uteruskontraktion, die als äußerst schmerzhaft empfunden werden können, machen das Wesen einer Dysmenorrhoe aus. *Vgl. z. B. J. Obstet. Gynäecol. Br. Commonw., Band 72, 1965, S. 185-192; V. R. Pickels et al. und J. Endocr., Band 77, 1978, S. 361-371; J. B. Maathuis et al.,* V. R. Pickels et al. beschreiben, daß unter Dysmenorrhoe leidende Patientinnen einen höheren Anteil an $PG_F$ produzieren. Daß die Gabe mäßiger Mengen von Progesteron dysmenorrhoeische Beschwerden steigern kann bzw. daß ein anovulatorischer Zyklus im allgemeinen mit schmerzlosen Blutungen endet stimme mit einer erhöhten Produktion der spasmogenen F-Prostaglandine durch das Endometrium überein. Die Autoren sehen - bis heute nicht verfügbare -spezifische "Antagonisten der Prostaglandine", wenn sie selektiv vom Myometrium aufgenommen würden, als nützlich für die Behandlung der Dysmenorrhoe an.

Es wurde nun gefunden, daß, entgegen der bisherigen Annahme, Antigestagene die uterine Produktion von Prostaglandinen (PG) hemmen.

Eine Hemmung der uterinen PG durch Antigestagene beseitigt oder verringert zumindest deutlich die Beschwerden, die mit einer Dysmenorrhoe einhergehen. Da die uterine PG-Synthese ganz wesentlich im Endometrium stattfindet, ist auch zu erwarten, daß Störungen und Beschwerden im Gefolge einer Endometriose durch Antigestagene ebenfalls günstig beeinflußt wird.

Dieser Effekt der Hemmung der uterinen Prostaglandinsynthese wurde an nichtgraviden Meerschweinchen beobachtet.

Die von uterinen PG am Ende des ca. 16-tägigen Zyklus erfolgende Luteolyse wurde von Antigestagenen blockiert. Es resultierte ein Verlauf der Serum-Progesteronwerte, wie er sonst nur in der Gravidität besteht. Eine Beeinträchtigung des normalen Zyklusgeschehens ist aber durch die gehemmte PG-Produktion nach Antigestagengabe beim Menschen nicht zu erwarten, da bei der Frau die Lebensdauer des Corpus luteum (stirbt am Zyklusende ab) nicht vom Uterus kontrolliert wird. Es ist aber zu erwarten, daß mit einer Hemmung der uterinen PG-Freisetzung eine Dämpfung der Uterusmotorik bei Vorliegen eine Dysmenorrhoe möglich ist.

Der Vorteil dieser Therapiemöglichkeit besteht darin, daß ein wünschenswertes Maß an Hemmung erreicht wird, ohne daß die PG-Funktion in anderen Organen beruhrt wird. Es wird ein hohes Maß an Organselektivität durch die Therapie der Dysmenorrhoe mit Antigestagenen erreicht.

Antigestagene können mit Beginn dysmenorrhoeischer Beschwerden, wie "Ziehen im Unterleib", angewendet werden. Es ist manchmal sinnvoll, den Beginn der Therapie auf etwas frühere Phasen der Lutealphase zu legen; um ein ausreichendes Maß an Hemmung zu erreichen.

Nach einer bevorzugten Ausführungsform wird die Antigestagenbehandlung in der Regel über 1 bis 6, vorzugsweise 1 bis 4 Tage, beginnend um den Menstruationszeitpunkt vorgenommen.

Die Antigestagene werden gemäß vorliegender Erfindung in Mengen von im allgemeinen 2 bis 50 mg, vorzugsweise 5 bis 20 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on pro Tag oder einer biologisch äquivalenten Menge eines anderen Antigestagens eingesetzt.

Als Antigestagene kommen alle Verbindungen infrage, die eine starke Affinität zum Gestagenrezeptor (Progesteronrezeptor) besitzen und dabei keine eigene gestagene Aktivität zeigen. Als kompetitive Progesteronantagonisten kommen beispielsweise folgende Steroide infrage:

11$\beta$-[(4-N,N-Dimethylamino)-phenyl]-17$\beta$-hydroxy-17$\alpha$-propinyl-4,9(10)-estradien-3-on,

11$\beta$-[(4-N,N-Dimethylamino)-phenyl]-17$\beta$-hydroxy-18-methyl-17$\alpha$-propinyl-4,9(10)-estradien-3-on und

11$\beta$[(4-N,N-Dimethylamino)-phenyl]-17 a$\beta$-hydroxy-17a$\alpha$-propinyl-D-homo-4,9(10)-16-estratrien-3-on

(Europäische Patentanmeldung 82400025.1 - Veröffentlichungsnummer 0 057 115),
11$\beta$-Methoxyphenyl-17$\beta$-hydroxy-17$\alpha$-ethinyl-4,9(10)-estradien-3-on (Steroids 37 (1981) 361-382),
11$\beta$-[(4-N,N-Dimethylamino)-phenyl]-17$\beta$-hydroxy-17$\alpha$-(hydroxyprop-1-(Z)-enyl)-4,9(10)-estradien-3-on
(Europäische Patentanmeldung 847300147.0 - Veröffentlichungsnummer 0 147 361),
11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on
(Europäische Patentanmeldung 84730062.1 - Veröffentlichungsnummer 0 129 499).

Die Antigestagene können zum Beispiel lokal, topisch, enteral oder parenteral appliziert werden.

Für die bevorzugte orale Applikation kommen insbesondere Tabletten, Dragées, Kapseln, Pillen, Suspensionen oder Lösungen infrage, die in üblicher Weise mit den in der Galenik gebräuchlichen Zusätzen und Trägersubstanzen hergestellt werden können. Für die lokale oder topische Anwendung kommen beispielsweise Vaginalzäpfen oder transdermale Systeme wie Hautpflaster infrage.

Eine Dosierungseinheit enthält etwa 2 bis 50 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on oder eine biologisch äquivalente Menge eines anderen Antigestagens.

Die nachstehenden Beispiele sollen die galenische Formulierung von Antigestagenen erläutern.

Beispiel 1

Zusammensetzung einer Tablette mit 10 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on
zur oralen Applikation

| | |
|---|---|
| 10,0 mg | 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on |
| 140,5 mg | Laktose |
| 69,5 mg | Maisstärke |
| 2,5 mg | Polyvinylpyrrolidon 25 |
| 2,0 mg | Aerosil |
| 0,5 mg | Magnesiumstearat |
| 225,0 mg | Gesamtgewicht |

Beispiel 2

Zusammensetzung einer öligen Lösung mit 50 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on
zur parenteralen Applikation

In je 1 ml Rizinusöl/Benzylbenzoat im Volumenverhältnis 6:4 werden 50 mg des Antigestagens gelöst.

Pharmakologische Beobachtungen

Nichtgravide Meerschweinchen erhielten täglich vom 8. bis 16. Tag des ca. 16-tägigen Zyklus 10 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on.

Die Testsubstanz wurde in 1 ml Benzylbenzoat/Rizinusöl (1:2) gelöst und subkutan injiziert. Täglich wurde der Progesterongehalt im Serum bestimmt.

Aus der folgenden Tabelle ist ersichtlich, daß die Serum-Progesteronwerte bei den mit dem Antigestagen behandelten Tieren ansteigen, während die Serum-Progesteronwerte der mit Lösungsmittel behandelten Kontrollen zum Zyklusende hin stark abfallen.

Entsprechende Befunde wurden dosisabhängig für diese Substanz und auch andere Antigestagene in analogen Versuchsanordnungen erhalten.

Der Anstieg von Progesteron im Blut von Tieren, die mit dem Antigestagen behandelt wurden, reflektiert eine Hemmung der uterinen PG-Freisetzung.

6

Behandlung nicht gravider Meerschweinchen mit 10 mg 11ß-[(4-N,N-Dimethylamino)-phenyl]-17α-hydroxy-17ß--(3-hydroxypropyl)-13α-methyl-4,9(10)-gonadien-3-on

$$\frac{\text{Progesteron} / \text{nmol} / 1 \quad \text{Serum}}{}$$

| Tier-Nr. | $d_0$ | $d_1$ | $d_4$ | $d_6$ | $d_8$ | $d_{10}$ | $d_{12}$ | $d_{14}$ | $d_{15}$ | $d_{16}$ | $d_{17}$ |
|---|---|---|---|---|---|---|---|---|---|---|---|
| 6637 | 1.3 | 23.2 | 7.2 | 10.2 | 19.9 | 20.5 | 18.8 | 10.2 | --- | 16.0 | 26.8 |
| 6638 | 1.9 | 1.5 | 8.5 | 11.9 | 8.4 | 14.3 | 16.1 | 17.4 | ---- | 25.6 | 24.6 |
| 6639 | 1.6 | 5.9 | 13.5 | 13.6 | 9.5 | 13.0 | 13.0 | 24.2 | 18.4 | --- | --- |
| 6640 | 0.4 | 4.8 | 8.2 | 17.6 | 22.2 | 23.6 | 36.0 | 31.4 | 39.8 | --- | --- |
| 6641 | o.4 | 6.1 | 5.3 | 7.1 | 8.0 | 9.5 | 10.6 | 5.2 | --- | 2.8 | 2.8 |
| 6642 | 3.7 | 2.3 | 11.7 | 8.7 | 18.1 | 5.3 | 1.3 | 0.8 | 1.1 | --- | --- |

Mit Antigestagen behandelte Tiere (6637–6640)

Mit Lösungsmittel behandelte Kontrollen (6641–6642)

## Patentansprüche

1. Verwendung von Antigestagenen für die Herstellung von Arzneimitteln zur Therapie und Prophylaxe dysmenorrhoeischer Beschwerden.

2. Verwendung von Antigestagenen für die Herstellung von Arzneitteln zur Behandlung der durch eine Endometriose hervorgerufenen Störungen und Beschwerden.

**3.** Verwendung von 11$\beta$-[(4-N,N-Dimethylamino)-phenyl]-17$\beta$-hydroxy-17$\alpha$-propinyl-4,9(10)-estradien-3-on, 11$\beta$-[(4-N,N-Dimethylamino)-phenyl-17$\beta$-hydroxy-18-methyl-17$\alpha$-propinyl-4,9(10)-estradien-3-on, 11$\beta$-[(4-N,N-Dimethylamino)-phenyl]-17a$\beta$-hydroxy-17a$\alpha$-propinyl-D-homo-4,9(10)-16-estratrien-3-on, 11$\beta$-Methoxyphenyl-17$\beta$-hydroxy-17$\alpha$-ethinyl-4,9(10)-estradien-3-on, 11$\beta$-[(4-N,N-Dimethylamino)-phenyl]-17$\beta$-hydroxy-17$\alpha$-(hydroxyprop-1-(Z)-enyl)-4,9(10)-estradien-3-on oder 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on als Antigestagene nach Anspruch 1 oder 2.

**Claims**

**1.** The use of antigestagens for the preparation of medicaments for the treatment and prophylaxis of dysmenorrhoeic complaints.

**2.** The use of antigestagens for the preparation of medicaments for the treatment of disorders and complaints caused by endometriosis.

**3.** The use of 11$\beta$-[(4-N,N-dimethylamino)-phenyl]-17$\beta$-hydroxy-17$\alpha$-propynyl-4,9(10)-oestradien-3-one, 11$\beta$-[(4-N,N-dimethylamino)-phenyl]-17$\beta$-hydroxy-18-methyl-17$\alpha$-propynyl-4,9(10)-oestradien-3-one, 11$\beta$-[(4-N,N-dimethylamino)-phenyl]-17a$\beta$-hydroxy-17a$\alpha$-propynyl-D-homo-4,9(10)-16-oestratrien-3-one, 11$\beta$-methoxyphenyl-17$\beta$-hydroxy-17$\alpha$-ethynyl-4,9(10)-oestradien-3-one, 11$\beta$-[(4-N,N-dimethylamino)-phenyl]-17$\beta$-hydroxy-17$\alpha$-(hydroxyprop-1-(Z)-enyl)-4,9(10)-oestradien-3-one or 11$\beta$-[(4-N,N-dimethylamino)-phenyl]-17$\alpha$-hydroxy-17$\beta$-(3-hydroxypropyl)-13$\alpha$-methyl-4,9(10)-gonadien-3-one as antigestagens according to claim 1 or 2.

**Revendications**

**1.** Utilisation d'antigestagènes pour la préparation de médicaments destinés à la thérapie et à la prophylaxie de troubles de dysménorrhée.

**2.** Utilisation d'antigestagènes pour la préparation de médicaments destinés au traitement des perturbations et troubles engendrés par une endométriose.

**3.** Utilisation de 11$\beta$-[(4-N,N-diméthylamino)-phényl]-17$\beta$-hydroxy-17$\alpha$-propynyl-4,9(10)-oestradién-3-one, de 11$\beta$-[(4-N,N-diméthylamino)-phényl]-17$\beta$-hydroxy-18-méthyl-17$\alpha$-propynyl-4,9(10)-oestradién-3-one, de 11$\beta$-[(4-N,N-diméthylamino)-phényl]-17a$\beta$-hydroxy-17a$\alpha$-propynyl-D-homo-4,9(10)-16-oestratrién-3-one, de 11$\beta$-méthoxyphényl-17$\beta$-hydroxy-17$\alpha$-éthynyl-4,9(10)-oestradién-3-one, de 11$\beta$-[(4-N,N-diméthylamino)-phényl]-17$\beta$-hydroxy-17$\alpha$-(hydroxyprop-1-(Z)-ényl-4,9(10)-oestradién-3-one ou de 11$\beta$-[(4-N,N-diméthylamino)-phényl]-17$\alpha$-hydroxy-17$\beta$-(3-hydroxypropyl)-13$\alpha$-méthyl-4,9(10)-gonadién-3-one comme antigestagènes suivant l'une des revendications 1 et 2.